# EUROPEAN PATENT SPECIFICATION

(11) **EP 2 050 411 B1**
(45) Date of publication and mention of the grant of the patent: **09.03.2011**
(21) Application number: 07790830.9
(22) Date of filing: 17.07.2007
(51) Int. Cl.: A61B 18/20, A61B 18/24, G01H 9/00, A61N 5/067, A61N 5/06, A61B 18/26, G01L 1/24, G01B 11/16, A61B 17/00, G02B 6/02, A61B 17/22

(54) **LIGHT IRRADIATION APPARATUS**
LICHTBESTRAHLUNGSGERÄT
APPAREIL D'IRRADIATION LUMINEUSE

(30) Priority: 07.08.2006 JP 2006214775
(43) Date of publication of application: 22.04.2009
(73) Proprietor: Hamamatsu Photonics K.K., Hamamatsu-shi, Shizuoka 435-8558 (JP)
(72) Inventor: YAMASHITA, Daisuke, Hamamatsu-shi Shizuoka 435-8558 (JP); YAMASHITA, Yutaka, Hamamatsu-shi Shizuoka 435-8558 (JP); YAMAUCHI, Toyohiko, Hamamatsu-shi Shizuoka 435-8558 (JP); OKADA, Hiroyuki, Hamamatsu-shi Shizuoka 435-8558 (JP)
(74) Representative: Grünecker, Kinkeldey, Stockmair & Schwanhäusser Anwaltssozietät
(86) International application number: PCT/JP2007/064068
(87) International publication number: WO 2008/018270

(56) References cited:
- WO-A-97/39690
- JP-A- 03 111 039
- JP-A- 03 505 683
- JP-A- 04 022 353
- JP-A- 2002 272 737
- JP-A- 2006 501 930
- US-A- 5 709 676
- US-A1- 2004 067 000

## Description

### Technical Field

The present invention relates to a light irradiation device for irradiating laser light from an optical fiber to an object formed inside a blood vessel such as a thrombus.

### Background Art

For the purpose of dissolving a thrombus formed inside a blood vessel, etc., there has been conventionally proposed laser light irradiated to an object such as a thrombus by using an optical fiber. In order to irradiate the laser light safely and effectively, it is necessary to make the leading end of the optical fiber accurately in contact with a thrombus. Since the laser light is used to effect thrombolysis in a state that a catheter is inserted into a human body, X-ray transmission by using a contrast medium for a blood vessel is absolutely necessary for confirming how the fiber for irradiating laser light is in contact with the thrombus.

On the contrary, as a method for detecting an irradiation state of the laser light inside a blood vessel (the presence of vapor bubbles), there is, for example, a method described in Patent Document 1. According to this method, after detection light is irradiated, a change in intensity of the detection light returned to an optical fiber by reflection and diffusion is detected, by which the above-described irradiation state of the laser light is detected.
Patent Document 1: Published Japanese Translation of PCT Application No. 2001-517805

US 2004/067000 A1 relates to an imaging apparatus comprising an elongated body having proximal and distal ends, the body including an optical fiber; an optical-to-acoustic transducer configured to generate acoustic energy for imaging a region near the distal end; an acoustic-to-optical transducer configured to sense acoustic energy from the region near the distal end; and a user interface.

WO 97/39690 A relates to an apparatus comprising a fiber optic for insertion into the vasculature to a point near an occlusion, wherein said fiber optic comprises a proximal end and a distal end; and a laser to provide laser light for coupling into said proximal end.

### Disclosure of the Invention

### Problem to be Solved by the Invention

There has been demanded a method for understanding more safely an irradiation state of the laser light since the above-described method for X-ray transmission places a burden on a patient.

However, according to the method described in Patent Document 1, no detection can be made, unless substances such as vapor bubbles that will change the properties of reflection and diffusion of light are in contact with the leading end of an optical fiber. Thus, the method described in Patent Document 1 cannot be applicable to a case where the substances such as vapor bubbles that will change the properties of reflection and diffusion of light are not generated or where the substances are not in contact with the emitting end face of the optical fiber even if they are generated. More specifically, the method described in Patent Document 1 can be used only in very limited cases.

The present invention has been made to solve the above-described problem, an object of which is to provide a light irradiation device capable of detecting an irradiation state of the laser light safely and more reliably on irradiation of laser light to an object.

### Means for Solving the Problem

In order to attain the above object, the light irradiation device includes a first light source for generating laser light, a second light source for generating detection light of a predetermined wavelength, an optical fiber for inputting the laser light on one end face from the first light source, emitting the laser light from the other end face to irradiate the laser light to an object, and inputting the detection light on the one end face from the second light source, wherein the optical fiber has a FBG (Fiber Bragg Grating) reflecting light of the predetermined wavelength adjacent to the other end face, reflection light detection means for detecting an intensity of the detection light input to the optical fiber on the one end face from the second light source, reflected by the FBG and emitted from the one end face, and irradiation state detection means for detecting an irradiation state of the laser light to the object from the optical fiber based on the intensity of the detection light detected by the reflection light detection means, wherein the laser light generated from the first light source is light other than light of a wavelength reflected by the FBG. This embodiment is not a part of the present invention.

In the light irradiation device laser light is generated from the first light source and emitted from the optical fiber. In response to the thus emitted light, pressure such as acoustic pressure acts on the vicinity of a position of light emitted from the optical fiber and the optical fiber is distorted at the position. Due to this distortion, the FBG formed on the optical fiber is changed in reflection characteristics, by which the thus reflected detection light is changed in intensity. Therefore, the light irradiation device detects the intensity of the detection light reflected by the FBG, thus making it possible to detect the irradiation state of the laser light from the optical fiber to the object based on the intensity.

As described above, according to the light irradiation device , detection light is used to detect an irradiation state of the laser light, thus making it possible to make the above detection safely. Further, in the light irradiation device , the detection light transmitted through an optical fiber is used to detect pressure acting on an optical fiber depending on emitted laser light, thereby detecting an irradiation state of the laser light. Therefore, in the light irradiation device, even in a general case where there are no such substances that will change the properties of reflection and diffusion of light, it is possible to detect the irradiation state of the laser light. More specifically, according to the light irradiation device it is possible to detect more reliably the irradiation state of the laser light.

Further, in order to attain the above object, the light irradiation device of the present invention includes a first light source for generating laser light, a second light source for generating detection light of a predetermined wavelength, a first optical fiber for inputting laser light on one end face from the first light source, emitting the laser light from the other end face to irradiate the laser light to an object, a second optical fiber for inputting the detection light on the one end face from the second light source, wherein the second optical fiber has a FBG reflecting light of the predetermined wavelength adjacent to the other end face of the first optical fiber, reflection light detection means for detecting the detection light input to the second optical fiber on the one end face from the second light source, reflected by the FBG and emitted from the one end face, and irradiation state detection means for detecting an irradiation state of the laser light to the object from the first optical fiber based on an intensity of the detection light detected by the reflection light detection means.

As with the above-described light irradiation device, since the thus constituted light irradiation device also detects an irradiation state of the laser light by using detection light, it is possible to make the detection safely. Further, in the light irradiation device of the present invention, the detection light transmitted through an optical fiber is used to detect pressure acting on the optical fiber depending on the emitted laser light, thereby detecting an irradiation state of the laser light. Therefore, in the light irradiation device of the present invention, even in a general case where there are no such substances that will change the properties of reflection and diffusion of light, it is possible to detect the irradiation state of the laser light. More specifically, according to the light irradiation device of the present invention, it is possible to detect more reliably the irradiation state of the laser light.

It is desirable that the irradiation state detection means detects the irradiation state based on a change in the intensity of the detection light detected by the reflection light detection means on irradiation of the laser light to the object. According to this constitution, pressure acting on an optical fiber can be reliably detected, thus making it possible to detect more reliably the irradiation state of the laser light.

It is desirable that the second light source generates light of plural wavelengths and a plurality of FBGs are formed so as to reflect the light of plural wavelengths. According to this constitution, detection light reflected by the FBG can be detected reliably, thus making it possible to detect more reliably an irradiation state of laser light.

It is desirable that the light irradiation device further includes a catheter for fixing the optical fiber through the optical fiber. It is also desirable that the light irradiation device further includes a catheter for fixing the first and second optical fibers through the first and second optical fibers. According to these constitutions, the present invention is reliably applicable to medical treatment of a thrombus and others.

### Effect of the Invention

According to the present invention, since detection light is used to detect an irradiation state of the laser light, it is possible to make the detection safely. Further, in the present invention, the detection light transmitted through an optical fiber is used to detect pressure acting on the optical fiber depending on the emitted laser light, thereby detecting the irradiation state of the laser light. Therefore, in the present invention, even in a general case where there are no such substances that will change the properties of reflection and diffusion of light, it is possible to detect the irradiation state of the laser light. More specifically, according to the present invention, it is possible to detect more reliably the irradiation state of the laser light.

### Brief Description of the Drawings

Fig. 1 is a drawing showing a constitution of the light irradiation device which is not an embodiment in the present invention.
Fig. 2 is a drawing showing sites at which a FBG of an optical fiber is formed. This is not an embodiment of the present invention.
Fig. 3 is a drawing showing another example of sites at which the FBG of the optical fiber is formed. This is not an embodiment of the present invention.
Fig. 4 is a drawing showing an optical fiber for laser light and an optical fiber for detection light.

### Description of Symbols

- 1:: light irradiation device
- 2:: blood vessel
- 3:: thrombus
- 10:: laser light source
- 20:: light source for detection light
- 30:: optical fiber
- 30a, 30b:: end face
- 32:: FBG
- 41, 42:: lens
- 43, 44:: beam splitter
- 50:: light detector
- 60:: signal processing unit
- 70:: catheter
- 80:: guide wire

### Best Modes for Carrying Out the Invention

Hereinafter, a description will be given in detail for an embodiment of the light irradiation device by referring to the drawings. It is noted that in describing the drawings, the same symbols are given to the same components, with overlapping description omitted. Further, the dimensional ratios of the drawings are not always in agreement with those described.

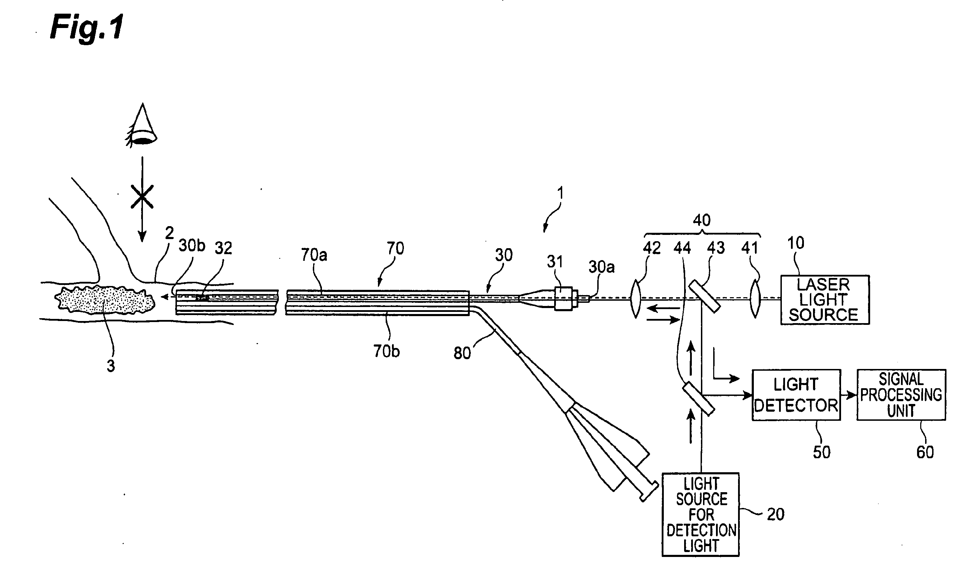

Fig. 1 shows a constitution of the light irradiation device 1 which is not a part of the present invention. The light irradiation device 1 is a device for giving medical treatment to a thrombus 3 formed inside a blood vessel 2 in a human body or others. More specifically, the light irradiation device 1 is that in which an optical fiber 30 is inserted into the blood vessel 2 to irradiate laser light from the optical fiber 30 to the thrombus (object) 3 formed inside the blood vessel 2, thereby dissolving the thrombus 3. Further, in the light irradiation device 1 of the present embodiment, in order to irradiate laser light safely and efficiently, detection is made for an irradiation state of the laser light. The irradiation state of the laser light means a relative position of the emitting end of laser light of the optical fiber 30 with respect to the thrombus 3 to be treated (whether or not irradiation is made from an appropriate position), irradiation effects of the laser light (whether or not the thrombus 3 is dissolved by the laser light) and the like.

Hereinafter, a description will be given for a constitution of the light irradiation device 1. As shown in Fig. 1, the light irradiation device 1 is constituted with a laser light source 10, a light source for detection light 20, an optical fiber 30, an optical system 40, a light detector 50, a signal processing unit 60 and a catheter 70.

The laser light source 10 is a first light source for generating laser light for irradiation to a thrombus 3. Laser light generated from the laser light source 10 is pulsed light (pulsed laser). The laser light source 10 is provided so that the generated laser light is input to the optical fiber 30. The laser light generated from the laser light source 10 is made incident into one end face 30a of the optical fiber 30 and input into the optical fiber 30. On this input, the laser light is collimated by a lens 41 included in the optical system 40 and made incident from a lens 42 into the end face 30a of the optical fiber 30.

It is preferable that laser light generated from the laser light source 10 is made appropriate in wavelength and intensity so as to dissolve the thrombus 3. Further, as will be described later, the laser light must be adjusted so that the wavelength thereof will not interfere with detection light generated from a light source for detection light 20. A laser diode, for example may be used as the laser light source 10.

The light source for detection light 20 is a second light source for generating detection light of a predetermined wavelength which is used in detecting an irradiation state of the laser light. The light source for detection light 20 is arranged, for example, in such a manner that detection light to be generated advances perpendicularly to an optical path of a laser beam from the laser light source 10. The detection light generated from the light source for detection light 20 is reflected by a beam splitter 43 included in an optical system 40 so that the optical path thereof is coincident with the optical path of laser light and advances to the optical fiber 30. Then, the detection light is made incident into one end face 30a of the optical fiber 30, as with laser light, and input into the optical fiber 30. On this input, the laser light is made incident into the end face 30a of the optical fiber 30 by the lens 42 included in the optical system 40.

For example, laser light is used as detection light generated from the light source for detection light 20. As will be described later, the detection light to be used is light of a wavelength which is reflected by a FBG 32. Further, the wavelength of the detection light is to be other than a wavelength (approximately ±10 nm) close to a wavelength of laser light so as to prevent interference with the laser light. For example, a laser diode is used as the light source for detection light 20.

The optical fiber 30 is that in which laser light is input from the laser light source 10 on one end face (laser-light incident end face) 30a and emitted from the other end face (laser-light emitting end face) 30b, thereby irradiating the light to the thrombus 3, which is an object.

When laser light is irradiated from the optical fiber 30 to the thrombus 3, the thrombus 3 absorbs energy and dissolves. In this instance, energy is absorbed into the thrombus 3 in very short time to generate an explosive sound and the optical fiber 30 is also subjected to physical pressure (acoustic pressure). Further, even where laser light is irradiated to blood and blood vessel walls other than the thrombus 3, the optical fiber 30 is subjected to pressure depending on an irradiation state of the laser light. It is noted that this irradiation is made in an invisible state when the optical fiber 30 is inserted into the blood vessel 2 from the laser-light emitting end face 30b side.

Further, the optical fiber 30 is that in which detection light is input from the light source for detection light 20 on one end face (detection-light incident end face) 30a. More specifically, two different types of light, or laser light and detection light, are made incident into the optical fiber 30. In this instance, the end face 30a, which is a laser-light incident end face and a detection-light incident end face of the optical fiber 30, is positioned and fixed so that light can be made incident reliably. For example, as shown in Fig. 1, a connector 31 is connected to the optical fiber 30 at the end portion of the end face 30a side. Then, the connector 31 is inserted into a predetermined position of a unit (not illustrated) in which the laser light source 10, the light source for detection light 20, the optical system 40 and others are accommodated, thereby positioning the end face 30a.

Still further, there is formed a FBG 32 for reflecting light of a predetermined wavelength adjacent (for example, a site of approximately several millimeters from the end face 30b) to the laser-light emitting end face 30b of the optical fiber 30. The wavelength of light reflected by the FBG 32 is a wavelength of detection light from the light source for detection light 20. Since laser light must be emitted from the laser-light emitting end face 30b, the laser light is light other than that of a wavelength reflected by the FBG 32. Therefore, the wavelength of laser light and that of detection light will differ from each other due to the above-described purpose, in addition to the prevention of interference.

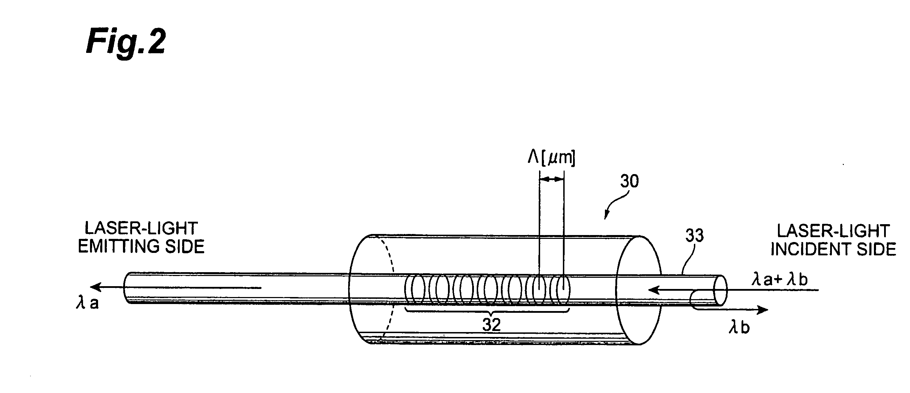

Fig. 2 shows a site at which a FBG 32 of the optical fiber 30 is formed. Laser light of a wavelength λa and detection light of wavelength λb are input to the optical fiber 30 from the incident side. The laser light of a wavelength λa is not reflected by the FBG 32 but transmitted, advancing to the emitting side of the laser light. On the other hand, the detection light of a wavelength λb is reflected by the FBG 32, advancing to the incident side.

As shown in Fig. 2, the FBG 32 is formed by imparting in a cyclic manner a strong and weak refractive index to the longitudinal direction (light axis direction) of a core 33 of the optical fiber 30. As a result, there is obtained a cyclic modulation: A in the longitudinal direction of the optical fiber 30, and only light of a wavelength: λb=2nΛ coincident with the cycle is reflected (in this instance, n denotes a refractive index in transmission mode). On the contrary, light of other wavelengths is transmitted, without detection of a change in refractive index. It is noted that normally light reflected by the FBG 32 constitutes not only light of the above-described wavelength λb but also a distribution of reflectance (reflection spectra) with respect to the wavelength axis at the center of the wavelength λb.

When the above-described pressure acts on the optical fiber 30, the optical fiber 30 is distorted. According to this distortion, the refractive index and the above-described A are changed, by which the wavelength of light reflected by the FBG 32 is shifted. More specifically, when the pressure acts on the optical fiber 30, there is found a change in reflection property of the FBG 32 (reflection spectra). Therefore, there is also found a change in reflectance with respect to detection light of a wavelength λb. Where the FBG 32 is set so as to reflect the detection light of a wavelength λb to a maximum extent (the reflectance is made greatest) in a state that no pressure acts on the optical fiber 30, the detection light of a wavelength λ b is made smaller in reflectance depending on the pressure on the optical fiber 30. More specifically, the intensity is decreased in the thus reflected detection light depending on the pressure on the optical fiber 30.

The FBG 32 is able to select freely a central wavelength and reflectance of the reflection spectrum, thus making it possible to form that which reflects detection light of any given wavelength. Further, it is also able to select freely the wavelength of detection light. Then, it is preferable that the wavelength of laser light appropriate for dissolving the thrombus 3 is first decided, and the wavelength of detection light and that of light reflected by the FBG 32 are decided thereafter so as not to interfere with laser light of the wavelength thereof. It is considered that light of 1/3, 1/5 and 1/7 wavelength... is also reflected in principle for the FBG 32. Therefore, the wavelength of detection light or that of light reflected by the FBG 32 is not to be set in the vicinity (approximately ±10nm) of laser light which is three times or five times ... in wavelength.

The optical system 40 is to guide laser light and detection light. More specifically, the optical system 40 is constituted with two lenses 41, 42 and two beam splitters 43, 44. These constituents are appropriately positioned and arranged so as to guide appropriately laser light and detection light. As described above, the optical system 40 allows the laser light from a laser light source 10 to be input into an optical fiber 30. Further, as described above, the optical system 40 allows the detection light from the light source for detection light 20 to be input into the optical fiber 30. Still further, the detection light reflected by the FBG 32 of the optical fiber 30 and emitted from the end face 30a is collimated by the lens 42, reflected by the beam splitter 43 and branched from an optical path of laser light (up to this stage, the optical path is the same as that of detection light made incident into the optical fiber 30). In addition, the thus emitted detection light is reflected by the beam splitter 44, branched from an optical path of the detection light made incident into the optical fiber 30 and made incident into a light detector 50.

It is noted that the optical system 40 does not necessarily require such a constitution as described above. Any optical system will do as long as it is that in which laser light and detection light are allowed to be input into the optical fiber 30 and the detection light reflected by the FBG of the optical fiber 30 is input into the light detector 50. For example, a circulator may be used to constitute an optical system having the above-described functions.

The light detector 50 is reflection light detection means for detecting an intensity of detection light input into the optical fiber 30, reflected by the FBG 32 and emitted from the end face 30a. The light detector 50 detects the intensity of each wavelength of detection light made incident. For example, a spectrum analyzer is specifically used as the light detector 50. Further, a photomultiplier, a photo diode or the like may be used other than the analyzer. The light detector 50 outputs a signal indicating the intensity of the thus detected light into a signal processing unit 60.

The signal processing unit 60 is irradiation state detection means for detecting an irradiation state of the laser light from the optical fiber 30 to an object based on the intensity of detection light detected by the light detector 50. The signal processing unit 60 is electrically connected to the light detector 50, receiving a signal indicating the intensity of detection light detected by the light detector 50 to process the signal, thereby detecting the irradiation state of the laser light to the object.

As described above, the intensity of detection light reflected by the FBG 32 is changed depending on the pressure acting on the optical fiber 30. Therefore, the change is evaluated to detect the irradiation state of the laser light. The change in intensity of the thus reflected detection light is evaluated by referring to rules stored in advance in the signal processing unit 60.

Where laser light is irradiated to a thrombus 3, explosive sound from the thrombus 3 gives physical pressure (acoustic pressure) to the optical fiber 30. Due to the influence of this pressure, there is found a change in reflection characteristics of the FBG 32. The change in reflection characteristics will accordingly result in a change in intensity of detection light detected by the FBG 32 and detected by the light detector 50. On the other hand, where laser light is irradiated to blood vessel walls or others which are free of blood, no explosive sound is generated. Further, where there is found a small quantity of blood in the above tissues, the explosive sound is decreased corresponding to the quantity of blood. Still further, there may be a difference in the explosive sound generated between the blood and the thrombus 3, that is, a difference in the method by which the pressure is given to the optical fiber 30.

With the above-described phenomena taken into account, rules are established in advance. For example, such rules are established that where the intensity of reflected detection light is decreased by more than a fixed ratio (specifically, where more than a fixed pressure is given to the optical fiber 30), laser light is used to detect dissolution of a thrombus 3. Further, such rules may be established that depending on the intensity (change) of reflected detection light, evaluation is made for a distance between an emitting end face 30b of the optical fiber 30 and the thrombus 3, which is an object. Still further, information on a change in intensity of detection light detected by the light detector 50 in itself may be used as information indicating an irradiation state of the laser light.

The information indicating the irradiation state of the laser light detected by the signal processing unit 60 is shown on a display (not illustrated) mounted on the light irradiation device 1 or output by voice through a speaker so as to be referred by a user. Further, the information indicating the irradiation state of the laser light may be used as reference information for irradiating subsequent laser light (feedback control).

More specifically, the signal processing unit 60 is provided with an AD converter for subjecting a signal from the light detector 50 to AD conversion and a constituent such as a CPU (central processing unit) for processing the above-described evaluation as data processing.

The catheter 70 is a flexible narrow tube and used to insert the optical fiber 30 into the blood vessel 2. The catheter 70 is provided with two holes 70a, 70b in the lengthwise direction (double lumen structure), and the optical fiber 30 is inserted into the one hole 70a of these for fixture. In this instance, the emitting end face 30b of laser light from the optical fiber 30 is to be located at the leading end of the catheter 70. This is for reliably irradiating laser light to the thrombus 3. The guide wire 80 is inserted into the other hole 70b for allowing the catheter to arrive at a target site. Further, physiological saline solution may be supplied from the other hole 70b.

Further, a description will be given for motions (method for using which is not a part of the invention) of the light irradiation device 1 of the present embodiment. As described above, the light irradiation device 1 of the present embodiment is to irradiate laser light to the thrombus 3 formed in the human blood vessel 2 and dissolve it. The catheter 70 is first inserted into the human blood vessel 2, thereby allowing it to advance to a site at which the thrombus 3 is formed. The catheter is inserted in such a direction that the emitting end face 30b of laser light from the optical fiber 30 is located forward.

When the catheter 70 substantially arrives at a site at which the thrombus 3 is formed, for the purpose of detecting an irradiation state of the laser light, detection light is generated from a light source for detection light 20 and made incident into the optical fiber 30. The detection light is reflected inside the optical fiber 30 by an FBG 32, emitted from the optical fiber 30 and input into the light detector 50. The intensity of detection light is detected by the light detector 50 and a signal indicating the intensity is input into a signal processing unit 60. The intensity of detection light reflected by the FBG 32 before irradiation of laser light is understood at the signal processing unit 60. The detection light is continuously made incident into the optical fiber 30.

Subsequently, laser light is generated from a laser light source 10 and made incident into the optical fiber 30. The laser light made incident into the optical fiber 30 is emitted from the emitting end face 30b into the blood vessel 2. As described above, there is found a change in pressure (acoustic pressure) acting on the optical fiber 30 depending on an irradiation state of the laser light to an object. Depending on the pressure acting thereon, there is also found a change in intensity of detection light reflected by the FBG 32 and detected by the light detector 50. The signal processing unit 60 compares the intensity of detection light before laser light is emitted from the laser light source 10 with that after it is emitted, thereby detecting the irradiation state of the laser light through evaluation of the change thereof. Information on the irradiation state of the thus detected laser light is referred by a user or used in feedback control of motions of the light irradiation device 1.

As described above, in the light irradiation device 1 of the present embodiment, detection light reflected by the FBG inside the optical fiber 30 is used to detect an irradiation state of the laser light. Therefore, it is possible to detect the irradiation state of the laser light without using a contrast medium for a blood vessel or irradiation of an X-ray. In other words, it is possible to make the detection safely.

Further, in the light irradiation device 1 of the present embodiment, the detection light transmitted through the optical fiber 30 is used to detect the pressure acting on the optical fiber 30 depending on the emitted laser light, thereby detecting an irradiation state of the laser light. Therefore, in the light irradiation device 1 of the present embodiment, even in a general case where there are no such substances that will change the properties of reflection and diffusion of light inside the blood vessel 2, it is possible to detect the irradiation state of the laser light. More specifically, according to the light irradiation device 1 of the present embodiment, it is possible to detect more reliably the irradiation state of the laser light.

Thereby, there is no risk that patients or operators are exposed to X-ray irradiation during medical treatment and the treatment can be conducted safely and accurately.

Further, in the light irradiation device 1 of the present embodiment, eliminated is a necessity for insertion into the blood vessel 2 exclusively for detecting an irradiation state of the laser light but only the FBG 32 may be formed at the optical fiber 30 for irradiation of laser light. The detection can be made only by mounting on a conventional device a light source for detection light 20, an optical system for detection light and devices 50, 60 for detecting detection light and analyzing it. Therefore, the light irradiation device can be made simple in constitution and easier in making the detection.

Still further, as described in the present embodiment, when the irradiation state is to be detected based on a change in intensity of detection light detected on irradiation of laser light to the thrombus 3, the pressure acting on the optical fiber 30 can be detected reliably and the irradiation state of the laser light can be detected more reliably. In addition, such a constitution is provided that is provided with a catheter 70 as with the present embodiment, thus making it possible to apply the device reliably to medical treatment of the thrombus 3.

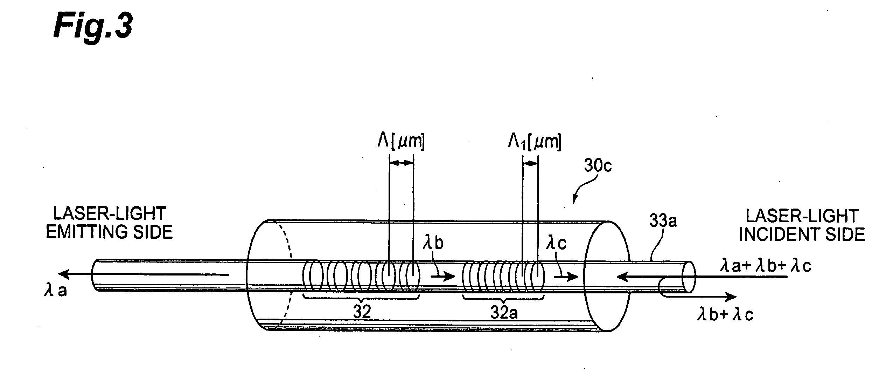

In the above-described embodiment, no particular description is given to the number of FBGs 32 formed. In addition to a single FBG, a plurality of FBGs may be formed which are adapted to reflect light of plural wavelengths which are different from each other. In such a constitution, light of plural wavelengths is to be generated accordingly from the light source for detection light 20. According to this constitution, since detection light reflected by the FBG 32 can be reliably detected, an irradiation state of the laser light can be detected more reliably. If the FBG is formed at every position of the optical fiber 30, it is possible to detect what kind of pressure acts on which position of the optical fiber 30. Thereby, the irradiation state can be detected in more detail. For example, as shown in Fig. 3, there may be used an optical fiber 30c in which FBGs 32, 32a are formed on a core 33a so as to reflect light of wavelength λb and that of wavelength λc, respectively, which are different from each other. In this instance, there is obtained a cyclic modulation: Λ₁ in the longitudinal direction of the optical fiber 30c at the FBG 32a, and only light of a wavelength: λc=2nΛ₁ coincident with the cycle is reflected (in this instance, n denotes a refractive index in transmission mode).

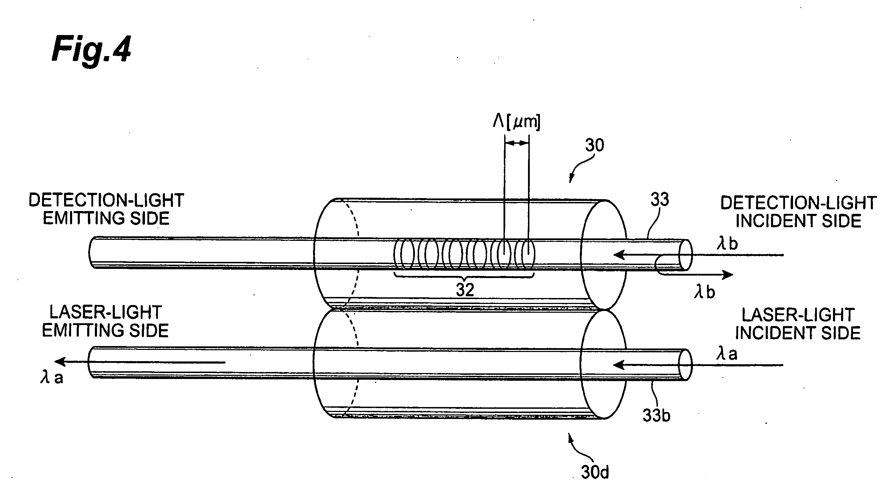

Further, the light irradiation device 1 of the present embodiment is constituted so as to be singular in the optical fiber 30. The device according to the present invention is, on the other hand, constituted so as to have two optical fibers, that is, an optical fiber for laser light (a first optical fiber) and an optical fiber for detection light (a second optical fiber). In that instance, the FBG is obviously formed only on the optical fiber for detection light. In this instance as well, the FBG is formed adjacent to an emitting end face of the optical fiber for laser light, for example, by bundling two optical fibers together so that the pressure resulting from irradiation of laser light to an object may act on the FBG For example, as shown in Fig. 4, the light irradiation device 1 may be provided with an optical fiber for laser light 30d and the optical fiber for laser light 30 in place of the optical fiber 30. In this instance, no FBG is formed on the optical fiber 30d. Laser light of wavelength λa is input from the incident side into the optical fiber 30d. Detection light of wavelength λb is input from the incident side into the optical fiber 30.

As described above, if laser light and detection light are to be input into different optical fibers, no interference will arise between each of the laser light and the detection light. Therefore, it is possible to make the laser light and the detection light equal in wavelength so that one light source can be used to supply both the laser light and the detection limt. This embodiment is not a part of the present invention.

Further, in the light irradiation device 1 of the present embodiment, laser light is to be irradiated to a thrombus 3 formed on the human blood vessel 2 but may be irradiated to others. The light irradiation device 1 may be applicable, for example, to that by which laser light is irradiated in order to break a calculus formed on the human urinary duct. More specifically, the present invention can be applicable to that by which laser light is irradiated to any object inside a blood vessel or the urinary duct in the human body or others. In that instance, it is desirable that the wavelength or intensity of laser light is changed appropriately, depending on an object, so as to obtain a sufficient irradiation effect of laser light.

## Claims

1. A light irradiation device comprising:
a first light source (10) for generating laser light;
and a first optical fiber (30d) for inputting the laser light on one
end face from the first light source, emitting the laser light from the other end face to irradiate the laser light to an object, **characterized in that** said device comprises a second light source (20) for generating detection light of a predetermined wavelength and a second optical fiber (30) for inputting the detection light on the one end face from the second light source, wherein the second optical fiber has a FBG (Fiber Bragg Grating, reflecting light of the predetermined wavelength adjacent to the other end face of the second optical fiber;
a reflection light detection means for detecting an intensity of the detection light input to the second optical fiber on the one end face from the second light source, reflected by the FBG and emitted from the one end face;
an irradiation state detection means adapted to detect an irradiation state of the laser light to the object from the first optical fiber based on the intensity of the detection light detected by the reflection light detection means;
wherein the laser light generated from the first light source is light of another wavelength than the wavelength reflected by the FBG,
wherein the irradiation state detection means (60) is adapted to detect to the irradiation state of the laser light based on a change in the intensity of the detection light, detected by the reflection light detection means, on irradiating the laser light to the object,
wherein the irradiation state of the laser light indicates a relative position of the emitting end of laser light of the optical fiber with respect to a thrombus (3) to be treated and irradiation effects of the laser light whether or not the thrombus (3) is dissolved by the laser light, and
wherein information on a change in intensity of detection light detected by a light detector (50) is used as information indicating an irradiation state of the laser light.

2. The light irradiation device according to claim 1, wherein the second light source (20) is adapted to generate light of plural wavelengths and a plurality of the FBGs are formed so as to reflect the light of plural wavelengths.

3. The light irradiation device according to any one of clams 1 to 2, further comprising a catheter (70), through which the optical fiber is insertable, for fixing the optical fiber.

4. The light irradiation device according to any one of claims 1 to 3, further comprising a catheter (70), through which the first and the second optical fibers are insertable, for fixing the first and the second optical fibers.

5. The light irradiation device according to any one of claims 1 to 4, wherein irradiation state detection means is adapted to evaluate, depending on the intensity of reflected detection light, a distance between an emitting end face of the optical fiber and the thrombus (3), which is an object.

## Patentansprüche

1. Lichtbestrahlungsvorrichtung, die umfasst:
eine erste Lichtquelle (10) zum Erzeugen von Laserlicht;
und eine erste Lichtleitfaser (30d) zum Eingeben des Laserlichtes an einer Endfläche von der ersten Lichtquelle, zum Emittieren des Laserlichtes von der anderen Endfläche, um das Laserlicht auf ein Objekt zu strahlen, **dadurch gekennzeichnet, dass** die Vorrichtung eine zweite Lichtquelle (20) zum Erzeugen von Erfassungslicht einer vorgegebenen Wellenlänge sowie eine zweite Lichtleitfaser (30) zum Eingeben des Erfassungslichtes an der einen Endfläche von der zweiten Lichtquelle umfasst, wobei die zweite Lichtleitfaser ein Bragg-Gitter (FBG) aufweist, das Licht der vorgegebenen Wellenlänge an die andere Endfläche der zweiten Lichtleitfaser angrenzend reflektiert;
eine Reflektionslicht-Erfassungseinrichtung zum Erfassen einer Stärke des Erfassungslichtes, das in die zweite Lichtleitfaser an der einen Endfläche von der zweiten Lichtquelle eingegeben wird, durch das Bragg-Gitter reflektiert wird und von der einen Endfläche emittiert wird;
eine Bestrahlungszustand-Erfassungseinrichtung, die so eingerichtet ist, dass sie einen Zustand der Laserlicht-Bestrahlung des Objektes über die erste Lichtleitfaser auf Basis der durch die Reflektionslicht-Erfassungseinrichtung erfassten Stärke des Erfassungslichtes erfasst;
wobei das von der ersten Lichtquelle erzeugte Laserlicht Licht einer anderen Wellenlänge als der durch das Bragg-Gitter reflektierten Wellenlänge ist,
die Bestrahlungszustand-Erfassungseinrichtung (60) so eingerichtet ist, dass sie den Laserlicht-Bestrahlungszustand auf Basis einer Änderung der durch die Reflektionslicht-Erfassungseinrichtung erfassten Stärke des Erfassungslichtes beim Bestrahlen des Objektes mit dem Laserlicht erfasst,
der Laserlicht-Bestrahlungszustand eine relative Position des Laserlicht emittierenden Endes der Lichtleitfaser in Bezug auf einen zu behandelnden Thrombus (3) und Bestrahlungseffekte des Laserlichtes dahingehend anzeigt, ob der Thrombus (3) durch das Laserlicht aufgelöst wird oder nicht, und
Informationen über eine Änderung der durch eine Lichterfassungseinrichtung (50) erfassten Stärke von Erfassungslicht als Informationen genutzt werden, die einen Laserlicht-Bestrahlungszustand anzeigen.

2. Lichtbestrahlungsvorrichtung nach Anspruch 1, wobei die zweite Lichtquelle (20) so eingerichtet ist, dass sie Licht mehrerer Wellenlängen erzeugt, und eine Vielzahl der Bragg-Gitter so ausgebildet sind, dass sie das Licht mehrerer Wellenlängen reflektieren.

3. Lichtbestrahlungsvorrichtung nach einem der Ansprüche 1 bis 2, die des Weiteren einen Katheter (70) umfasst, über den die Lichtleitfaser eingeführt werden kann, um die Lichtleitfaser zu fixieren.

4. Lichtbestrahlungsvorrichtung nach einem der Ansprüche 1 bis 3, die des Weiteren einen Katheter (70) umfasst, über den die erste und die zweite Lichtleitfaser eingeführt werden können, um die erste und die zweite Lichtleitfaser zu fixieren.

5. Lichtbestrahlungsvorrichtung nach einem der Ansprüche 1 bis 4, wobei die Bestrahlungszustand-Erfassungseinrichtung so eingerichtet ist, dass sie in Abhängigkeit von der Stärke von reflektiertem Erfassungslicht einen Abstand zwischen einer emittierenden Endfläche der Lichtleitfaser und dem Thrombus (3) bewertet, der ein Objekt ist.

## Revendications

1. Dispositif d'irradiation lumineuse comprenant :
une première source lumineuse (10) pour produire une lumière laser ;
et une première fibre optique (30d) pour l'entrée de la lumière laser sur une face d'extrémité, à partir de la première source lumineuse, et pour l'émission de la lumière laser, à partir de l'autre face d'extrémité, vers un objet,
**caractérisé en ce que** le dispositif comprend une seconde source lumineuse (20) pour produire une lumière de détection d'une longueur d'onde prédéterminée, et une seconde fibre optique (30) pour l'entrée de la lumière de détection, sur une face d'extrémité, à partir de la seconde source lumineuse, la seconde fibre optique ayant un réseau FBG (réseau de Bragg sur fibre) qui réfléchit la lumière de la longueur d'onde prédéterminée près de l'autre face d'extrémité de la seconde fibre optique ;
des moyens de détection de lumière de réflexion pour détecter une intensité de la lumière de détection entrée dans la seconde fibre optique, sur une première face, à partir de la seconde source lumineuse, réfléchie par le réseau FBG et émise à partir d'une face d'extrémité ;
des moyens de détection d'état d'irradiation aptes à détecter un état d'irradiation de la lumière laser sur l'objet à partir de la première fibre optique, sur la base de l'intensité de la lumière de détection détectée par les moyens de détection de lumière de réflexion ;
étant précisé que la lumière laser produite à partir de la première source laser est la lumière d'une longueur d'onde différente de la longueur d'onde réfléchie par le réseau FBG,
que les moyens de détection d'état d'irradiation (60) sont aptes à détecter l'état d'irradiation de la lumière laser sur la base d'un changement d'intensité de la lumière de détection, détectée par les moyens de détection de lumière de réflexion, lors de l'irradiation de l'objet par la lumière laser ;
que l'état d'irradiation de la lumière laser indique une position relative de l'extrémité émettrice de lumière laser de la fibre optique par rapport à un thrombus (3) à traiter, et les effets d'irradiation de la lumière laser, que le thrombus (3) soit dissous par la lumière laser ou pas, et
que des informations sur un changement d'intensité de la lumière de détection détectée par un détecteur de lumière (50) sont utilisées comme informations indiquant un état d'irradiation de la lumière laser.

2. Dispositif d'irradiation lumineuse selon la revendication 1, dans lequel la seconde source lumineuse (20) est apte à produire une lumière de plusieurs longueurs d'onde, et plusieurs réseaux FBG sont formés de manière à réfléchir la lumière de plusieurs longueurs d'onde.

3. Dispositif d'irradiation de lumière selon l'une quelconque des revendications 1 à 2, comprenant également un cathéter (70) dans lequel la fibre optique peut être insérée pour être fixée.

4. Dispositif d'irradiation de lumière selon l'une quelconque des revendications 1 à 3, comprenant également un cathéter (70) dans lequel les première et seconde fibres optiques peuvent être insérées pour être fixées.

5. Dispositif d'irradiation de lumière selon l'une quelconque des revendications 1 à 4, dans lequel des moyens de détection d'état d'irradiation sont aptes à évaluer, en fonction de l'intensité de la lumière de détection réfléchie, une distance entre une face d'extrémité émettrice de la fibre optique et le thrombus (3), qui est un objet.
